# EUROPEAN PATENT SPECIFICATION

(11) **EP 3 230 721 B1**
(45) Date of publication and mention of the grant of the patent: **03.06.2020**
(21) Application number: 15880472.4
(22) Date of filing: 30.01.2015
(51) Int. Cl.: G01N 27/06, G01N 27/07, B81B 7/00, B81B 1/00, B01L 3/00, G01N 33/487, H02M 3/155

(54) **DIAGNOSTIC CHIP**
DIAGNOSTISCHER CHIP
PUCE DE DIAGNOSTIC

(43) Date of publication of application: 18.10.2017
(73) Proprietor: Hewlett-Packard Development Company, L.P., Spring TX 77389 (US)
(72) Inventor: VALENCIA, Melinda M., San Diego, California 92127-1899 (US); LU, Sirena, San Diego, California 97330-4239 (US); DOMINGUE, Chantelle Elizabeth, Corvallis, Oregon 97330-4239 (US); SELLS, Jeremy, Corvallis, Oregon 97330-4239 (US); GIRI, Manish, Corvallis, Oregon 97330-4239 (US)
(74) Representative: Haseltine Lake Kempner LLP
(86) International application number: PCT/US2015/013731
(87) International publication number: WO 2016/122577

(56) References cited:
- WO-A1-2014/160513
- US-A1- 2005 009 101
- US-A1- 2010 075 340
- US-A1- 2010 276 017
- US-A1- 2012 142 032
- US-A1- 2013 288 386
- US-A1- 2013 295 588
- US-B1- 7 619 739

## Description

### BACKGROUND

Infectious diseases and other medical conditions affect human life on a continual basis. Developments have been made to detect the presence of, for example, antigens in blood or other bodily fluids in order to diagnose a patient's illness. In some cases, a microfluidic chip is used to analyze an analyte. US2013/295588A1 is prior art.

### BRIEF DESCRIPTION OF THE DRAWINGS

The accompanying drawings illustrate various examples of the principles described herein and are a part of the specification. The illustrated examples are given merely for illustration, and do not limit the scope of the claims.
Fig. 1 is a diagram of a microfluidic diagnostic chip incorporated into a cassette for analyzing an analyte within a fluid according to one example of the principles described herein.
Fig. 2 is a block diagram of a microfluidic diagnostic chip for analyzing an analyte within a fluid according to one example of the principles described herein.
Fig. 3 is a block diagram of a microfluidic diagnostic chip for analyzing an analyte within a fluid according to another example of the principles described herein.
Figs. 4A and 4B show block diagrams of two alternative MDC configurations according to two other examples of the principles described herein.
Fig. 5 is a flowchart showing a method of removing background noise from an impedance value of an analyte in a microfluidic diagnostic chip according to one example of the principles described herein.
Fig. 6 is a block diagram of a microfluidic diagnostic chip system (500) according to one example of the principles described herein.

Throughout the drawings, identical reference numbers designate similar, but not necessarily identical, elements.

### DETAILED DESCRIPTION

As mentioned above, microfluidic chips such as microfluidic diagnostic chips (MDCs) are used to help analyze fluid samples such as blood. In some MDCs, a sensor may be used to detect the impedance of the fluid as it is passed over the sensor. The fluid may include a number of particles in it that, when detected, help form the basis of a diagnosis. For example, a blood sample may include a number of different cells suspended in a fluid such as blood plasma and water. Additionally, the blood may include a number of analytes therein and may be mixed with, for example, reagents before being passed over the sensor. Consequently, the sensor is reading the impedance value the blood cells or analytes in the fluid as well as the fluid in which the blood cells are carried. The impedance value that the fluid contributes to the total impedance value may skew the results of a diagnosis or may lead to a misdiagnosis entirely.

The present specification, describes, in some examples, a microfluidic diagnostic chip comprises a number of input microfluidic channels, a number of output microfluidic channels, a number of interposing microfluidic channels fluidly coupled at one end to at least one input microfluidic channel and at a second end to at least one output microfluidic channel, and first and second microfluidic sensors within the number of interposing microfluidic channels wherein the microfluidic diagnostic chip is to compare output from the first microfluidic sensor sensing a fluid including an analyte to output from the second microfluidic sensor sensing the fluid lacking the analyte.

The present specification further describes method of removing background noise from an impedance value of an analyte in a microfluidic diagnostic chip, including measuring, with a first sensor, the impedance value of a fluid comprising an analyte, measuring, with a second sensor, the impedance value of the fluid without the analyte, and comparing, with a differential amplifier, the impedance value detected by the first sensor to the impedance value detected by the second sensor.

Further, the present specification describes a microfluidic chip, comprising a first sensor and a second sensor and a differential amplifier wherein the first sensor measures an impedance value of a fluid comprising an analyte, wherein the second sensor measures an impedance value of the fluid without the analyte, and wherein the differential amplifier compares the impedance values received from the first and second sensors.

In the present specification and in the appended claims, the term "fluid" is meant to be understood broadly as any substance that continually deforms (flows) under an applied shear stress. In one example, a fluid includes an analyte. In another example, a fluid includes a reagent.

Additionally, as used in the present specification and in the appended claims, the term "analyte" is meant to be understood as any substance that may be placed in a microfluidic diagnostic chip (MDC) to be analyzed. In one example, the analyte may be any bodily fluid such as, but not limited to, blood, urine, feces, mucus, or saliva.

Further, in the present specification and in the appended claims the term "reagent" is meant to be understood as a substance or compound that is added to a system in order to bring about a chemical reaction, or added to see if a reaction occurs. A reactant is meant to be understood as a substance that is consumed in the course of a chemical reaction. In one example, the reagent may be included within a fluid.

Still further, in the present specification and in the appended claims the term "analyte" is meant to be understood as any substance within a fluid that may be placed in a microfluidic diagnostic chip (MDC) to be analyzed. In one example, the analyte may be a number of particles within the fluid such as a blood cell. In this example, a specific type of blood cell is an analyte because it is to be analyzed by the MDC.

Even further, as used in the present specification and in the appended claims, the term "a number of" or similar language is meant to be understood broadly as any positive number comprising 1 to infinity.

In the following description, for purposes of explanation, numerous specific details are set forth in order to provide a thorough understanding of the present systems and methods. It will be apparent, however, to one skilled in the art that the present apparatus, systems and methods may be practiced without these specific details. Reference in the specification to "an example" or similar language means that a particular feature, structure, or characteristic described in connection with that example is included as described, but may not be included in other examples.

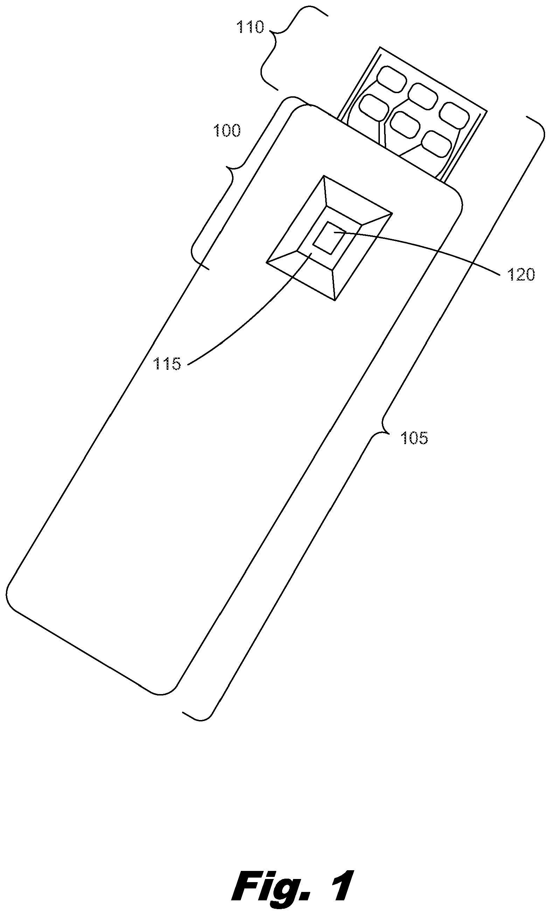

Turning now to the figures, Fig. 1 is a diagram of a microfluidic diagnostic chip (100) incorporated into a cassette (105) for analyzing an analyte according to one example of the principles described herein. In the example shown in Fig. 1, the MDC (100) forms part of a cassette (105). The cassette (105) further includes an electronic device interface (110). The interface may allow the MDC (100) to receive instructions and power from an external source such as a computing device. In this example, the MDC (100) forms the part of the cassette (105) that receives a fluid including an analyte while the cassette (105) and electronic device interface (110) provide the physical body to house the MDC and the power and instructions to operate the MDC respectively.

The cassette (105) may serve as a housing into which the MDC (100) and electronic device interface (110) are housed and protected from contamination and damage. The cassette (105) may also serve as a structure onto which a user may apply pressure in order to connect the electronic device interface (110) to an electronic device, for example directly to a computing device or to a connector that can be attached to a computing device..

The electronic device interface (110) may be any electrical contact points that may interface with an input/output port of an electronic device. In one example, the electronic device interface (110) is a universal serial bus (USB) interface capable of electrically coupling to a USB port in an electronic device. In other examples, the electrical contact points of the electronic device interface (110) may fit into a PCI bus, a PCIE bus, a SAS bus, and a SATA bus, among others. In one example, the electronic device interface (110) may include electrical contact points that interface with a specialized port in a specialized computing device.

The MDC (100) may include a feed tray (115) into which a fluid including an analyte is placed. The feed tray (115) directs the fluid into a fluidic slot (120) of the MDC (100). During operation, the fluid is placed in the feed tray (115) and passed into the fluidic slot (120). When the fluid is in the fluidic slot (120) the MDC (100) receives electrical power from an electrical device via the electronic device interface (110). In one example, the MDC (100) may further includes a differential amplifier to receive at least two signals from at least two sensors, compare the signals, and provide an output describing that comparison. In another example, the differential amplifier may be located off of the cassette (105) on, for example, a computing device electrically coupled to the cassette (105) via the electronic device interface (110).

The MDC (100) may further include a number of sensors located in a number of microfluidic channels defined in the MDC (100). In one example, the sensors are electrical impedance sensors capable of measuring an impedance value of a fluid including an analyte as the fluid is presented over the sensor. These sensors may measure the impedance of the fluid over time as well as measure the changes of the impedance value over time.

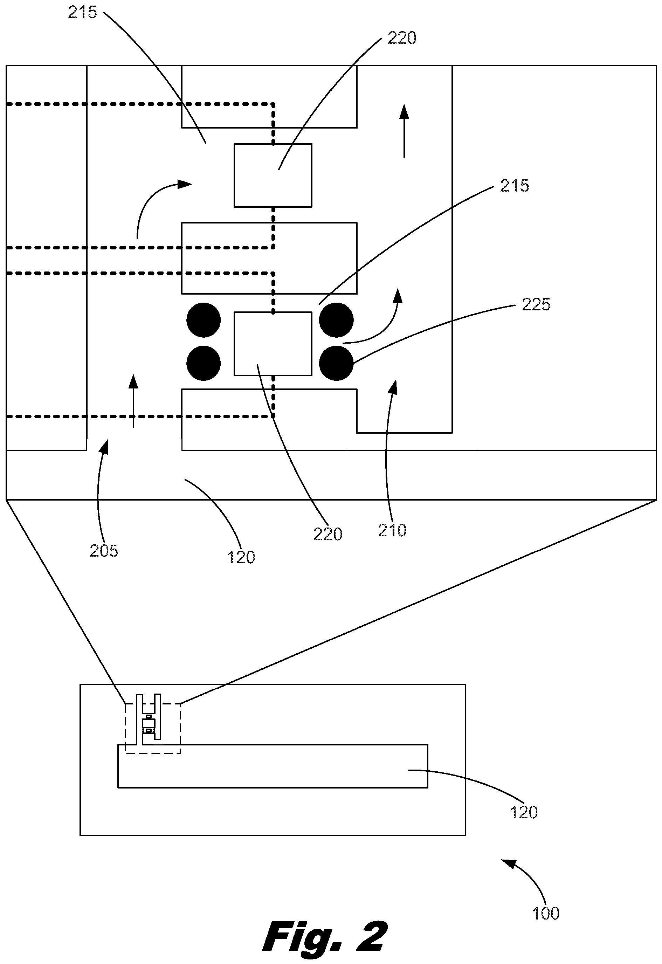

Fig. 2 is a block diagram of a microfluidic diagnostic chip (100) according to the invention for analyzing an analyte according to one example of the principles described herein. The MDC (100) may include a fluidic slot (120), an input microfluidic channel (205), an output microfluidic channel (210), a number of interposing microfluidic channels (215), a plurality of sensors (220) in the interposing microfluidic channels (215), and a number of particle blocking devices (225).

The fluidic slot (120) may be fluidly coupled to at least the input microfluidic channel (205) and may be defined into the substrate of which the MDC (100) is built. In one example, the substrate is made of silicon and the fluidic slot (120) is created by, for example, via sand blasting, laser etching, dry etching, wet etching, or combinations thereof. In one example, a fluid including the analyte to be analyzed by the MDC (100) may be fed into the fluidic slot (120) via the feed tray (115) using gravity. Additionally, the fluidic slot (120) may fill up with fluid before spilling over into the input microfluidic channel (205). In this example, the input microfluidic channel (205) is fluidly coupled to an upper portion of the fluidic slot (120).

Although Fig. 2 shows a single input microfluidic channel (205) any number of input microfluidic channels (205) may be used. In one example, a number of input microfluidic channels (205) may branch off from the fluidic slot (120) and may each be fluidly connected to their respective interposing microfluidic channels (215) and output channels (210). Consequently, the present specification contemplates the use of any number of these channels (205, 210, 215) to analyze a fluid and an analyte according to the principles described herein.

The input microfluidic channel (205) and output microfluidic channel (210) may each receive an amount of the fluid and pass it through the MDC (100) as indicated by the fluid flow arrows shown in Fig. 2. In one example, each of these channels (205, 210) may have a width of 6-10 µm. In another example, the channels may have a width of 1-100 µm. In still another example, the width of the input and output microfluidic channels (205, 210) is 10 µm. In yet another example, the width of the input microfluidic channel (205) is variable along the flow path of the fluid such that the proximal end of the input microfluidic channel (205) coupled to the fluidic slot (120) is wider than a remaining portion of the input microfluidic channel (205).

The input and output microfluidic channels (205, 210) may be fluidly coupled together via a number of interposing microfluidic channels (215). Although Fig. 2 shows two interposing microfluidic channels (215), any number of interposing microfluidic channels (215) may be used and the present specification contemplates the used of two, three, or more interposing microfluidic channels (215) without going beyond the principles described herein.

The interposing microfluidic channels (215) in Fig. 2 run parallel to each other. Each of the interposing microfluidic channels (215) includes a sensor (220) that reads an impedance value of the fluid in intimate contact with the sensory (220). In a first interposing microfiuidic channel (215), the fluid including the analyte is passed through. In this first interposing microfluidic channels (215), all particles, reagents, and other fluids originally present in the fluid are passed through. The sensor (220) in the first interposing microfluidic channel (215) then measures the impedance of that fluid including the analyte and sends an appropriate signal to a differential amplifier.

Concurrently, another sensor (220) located in a second interposing microfluidic channel (215) may detect the impedance value of only those portions of the fluid that can pass through the number of particle blocking devices (225). The particle blocking devices (225) may block certain particles or analytes within the fluid, preventing them from entering the second interposing microfluidic channel (215). In one example, the particle blocking devices (225) may include a number of micrometer sized pillars defined in the second interposing microfluidic channel (215). These pillars may be set close enough to each other to prevent the passage of any particular size of particle or analyte within the fluid. In one example, the particle blocking devices (225) may be so sized and arrange so as to permit the passage of some sizes of particles while block the passage of others. In another example, the particle blocking devices (225) may be so sized and arrange so as to block all particles or analyte in the fluid from passing through the second interposing microfluidic channel (215) and only allow the fluid to pass through. In the present specification, therefore, those particles and fluid allowed to pass through the second interposing microfluidic channel (215) may be referred to as a background solution.

The sensor (220) within the second interposing microfluidic channel (215) may also measure the impedance value of the background solution allowed to pass over the sensor (220). An electrical signal indicating the impedance value of the background solution is also passed from the sensor to the differential amplifier to be compared with the signal received from the sensor in the first interposing microfluidic channel (215).

During operation, the differential amplifier may receive the two signals and subtract the impedance value detected by the sensor (220) in the first interposing microfluidic channel (215) from the impedance value detected by the sensor in the second interposing microfluidic channel (215). Consequently, the true impedance value of the particles of interest within the analyte may be determined without the impedance value of the background solution contributing to the sensed impedance.

In one example, the impedance value of the respective constituents of the fluid presented over the two sensors (220) is measured at the same time. In another example, the impedance value of the respective constituents of the fluid presented over the two sensors (220) is measured at or around the same period of time. In yet another example, the impedance value of the respective constituents of the fluid presented over the two sensors (220) is measured as an analog value and presented to the differential amplifier as an analog signal. In this example, the differences in the values may be compared in real time as the fluid is passed through the MDC (100). In still another example, the analog signals received from the sensors (220) may be passed through the differential amplifier and the output may be passed through an analog to digital converter to produce a digital signal.

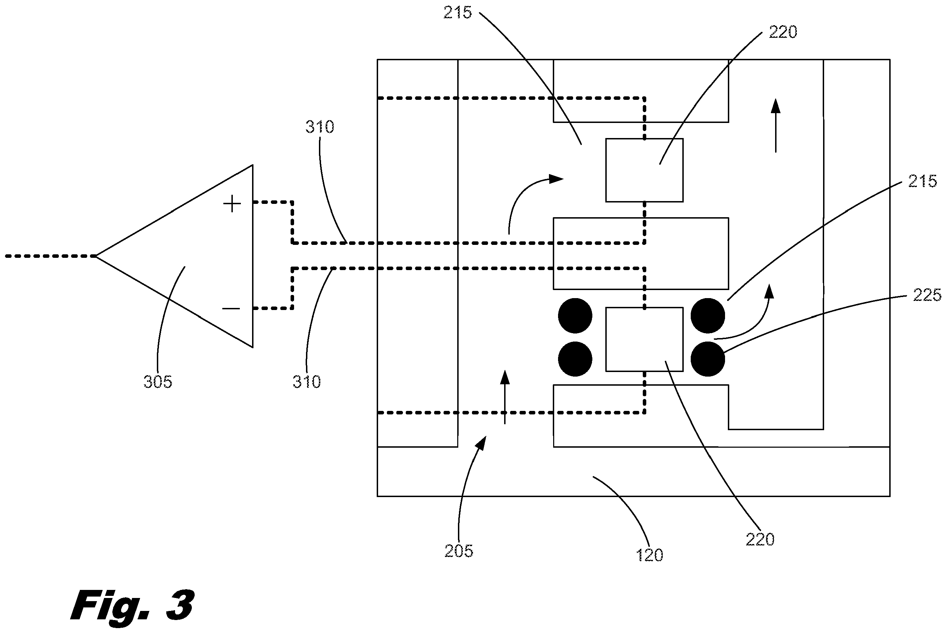

Fig. 3 is a block diagram of a microfluidic diagnostic chip (100) according to the invention for analyzing an analyte according to another example of the principles described herein. The MDC (100) shown in Fig. 3 includes similar elements as described in connection with Fig. 2 with the addition of a differential amplifier (305). As shown in Fig. 3, a number of electrical leads (310) may connect the sensors within the MDC (100) to the differential amplifier (305) so that the impedance signals detected by the sensors (220) may be fed into the differential amplifier (305). In one example, the differential amplifier (305) may form a part of the MDC (100) may be defined in the substrate of the MDC (100). In another example, the differential amplifier (305) may form part of the cassette (Fig. 1, 105) but not necessarily form part of the MDC (100). In this example, the signals presented to the differential amplifier (305) from the MDC (100) may be routed out of the MDC (100) and to the differential amplifier (305) via a number of electrical lines. In still another example, the differential amplifier (305) may for part of a separate computing device and may be electrically connected to the electrical leads (310) of the sensors (220) via the electronic device interface (Fig. 1, 110).

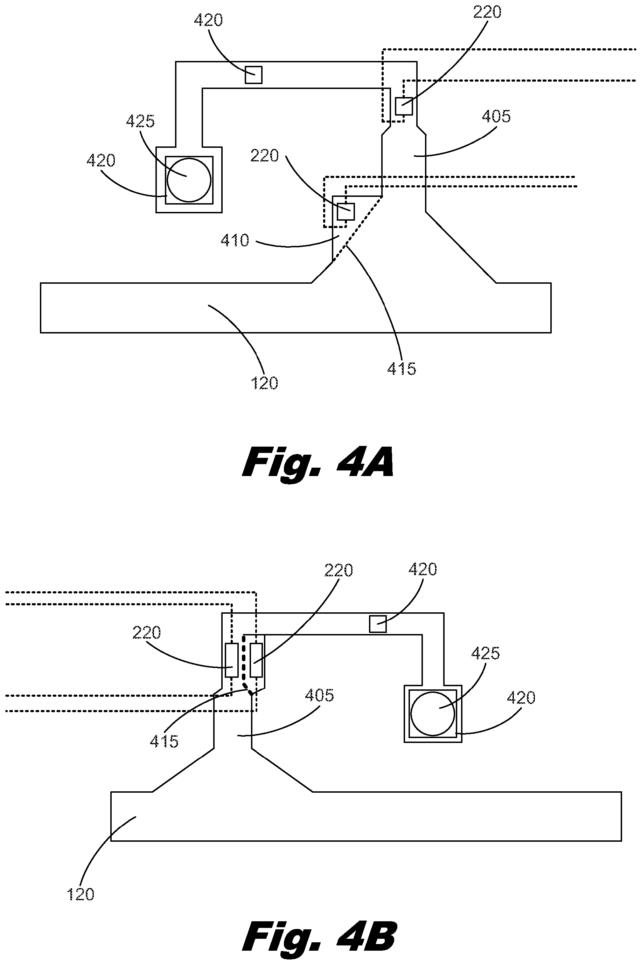

Figs. 2 and 3 both show the sensors (220) physically in parallel and within two physically parallel interposing microfluidic channels (215). Figs. 4A and 4B show block diagrams of two alternative MDC (100) configurations not according to the invention according to two other examples of the principles described herein. Fig. 4A shows a single main microfluidic channel (405) fluidically coupled to the fluidic slot (120). A channel cutout (410) is formed into a portion of the main microfluidic channel (405) and a particle blocking barrier (415) isolates the channel cutout (410) from the particles or analyte within the fluid as descried above. The particle blocking barrier (415) may function similar to the particle blocking devices (Fig. 2, 225) described in Fig. 2 by blocking those particles in the fluid that are not to be passed across the sensor (220) behind the barrier (415). In this example shown in Fig. 4A, a portion of the side wall of the main microfluidic channel (405) provides a barrier as well.

The sensors (220) in Fig. 4A are not arranged in a parallel configuration but are instead in a serial configuration where the sensor (220) behind the barrier (415) reads the impedance value of the fluid without the presence of the analyte before the second sensor (220) reads the impedance value of the entire fluid comprising the analyte. In this configuration, the signals sent to the differential amplifier (Fig. 3, 305) may be coordinated such that the signals from the first sensor behind the barrier (415) are delayed by some time period. This may be done to accommodate for any variations in particle or analyte density in the fluid.

Fig. 4A further includes a resistor (420) that serves to push or pull the fluid through the main microfluidic channel (405) defined in the MDC (100). During operation of the MDC (100) the fluid may be introduced into a fluidic slot (120). The fluid may then flow into the main microfluidic channel (405). The resistor (420) pushes or pulls the fluid through the main microfluidic channel (405) by superheating the fluid to create the drive bubble which grows and collapses in approximately 10 µs. The rapid bubble formation and collapse causes net flow of fluid within the main microfluidic channel (405) due to, in one example, the asymmetry in the fluidic design of the microfluidic channels (120, 405).

In one example, the resistor (420) formed in the microfluidic channel (405) has a length ranging from 10-110 µm and a width ranging from 10-100 µm. In another example, the resistor (420) is about 25 µm square. In one example, each pump (112) may include a thin film resistor. The thin film resistor may be made of tantalum or tantalum aluminum. In one example, the thickness of the resistor may be approximately 500 angstroms to 5000 angstroms. The resistor may be encapsulated with a passive film which is then encapsulated with a cavitation film. In one example, the passive film may be made of SiC or SiN and may be approximately 500-2000 angstroms thick. In another example, the cavitation film may be made of tantalum or platinum and may be approximately 500-2000 angstroms thick.

The MDC (100) of Fig. 4A further shows a bore (425) that serves as a hole through which an amount of fluid in the MDC (100) is ejected out of the main microfluidic channel (405) defined in the MDC (100). A resistor (420) is placed coplanar to the bore (425) such that when the resistor (420) has a voltage applied to it, a bubble forms. The bubble helps to push out an amount of the fluid from the MDC (100) and into a reservoir fluidly coupled to the bore (425). The fluid reservoir may be a dedicated reservoir in the MDC (100) or in the cassette (105) used to receive an amount of disposed fluid ejected from the bore (425).

Fig. 4B is a second example showing the sensors (220) in parallel and in contrast to that of Fig. 4A. In this example, the fluid is still passed through a single main microfluidic channel (405) but the sensors (220) analyze the fluid and analyte while being physically in parallel to each other. This allows for a single main microfluidic channel (405) to be used while still measuring the differences in impedance values of the same analyte concentration of fluid.

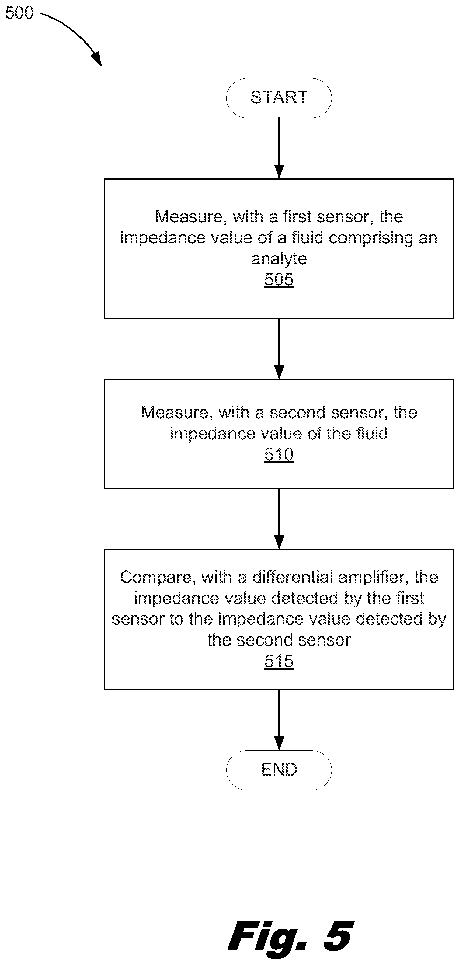

Fig. 5 is a flowchart showing a method of removing background noise from an impedance value of an analyte in an MDC (100) according to one example of the principles described herein. The method may begin with measuring (505), with a first sensor (220), the impedance value of a fluid comprising an analyte. As described above, the fluid may be passed through a microfluidic channel (215, 405) with a sensor therein.

The method (500) may continue with measuring (510), with a second sensor, the impedance value of the fluid without the analyte. As described above this is accomplished by providing a barrier (225, 415) or series of barriers (225, 415) that prevents the particles of the analyte from reaching the second sensor (220). In one example, the second sensor (220) is located in a separate microfluidic channel (215, 405) from that of the first sensor (220). In another example, the second sensor (220) and first sensor (220) are located in the same microfluidic channel (215, 405). In still another example, the second sensor (220) and first sensor (220) are located in the same microfluidic channel (215, 405) and the microfluidic channels (205, 210, 215) are designed such that the fluid including the analyte is recirculated over the first and second sensors (220). In this example, the recirculation of the fluid may allow for a reaction to take place between, for example, the analyte and a reagent. Here, the sensors may further measure the changing impedance value of the analyte and reagent mixture as a reaction is progressing over time.

The method (500) may continue with comparing, with a differential amplifier (305), the impedance value detected by the first sensor (220) to the impedance value detected by the second sensor (220). The comparison may include subtracting the impedance value sensed by the second sensor (220) from the impedance value sensed by the first sensor (220).

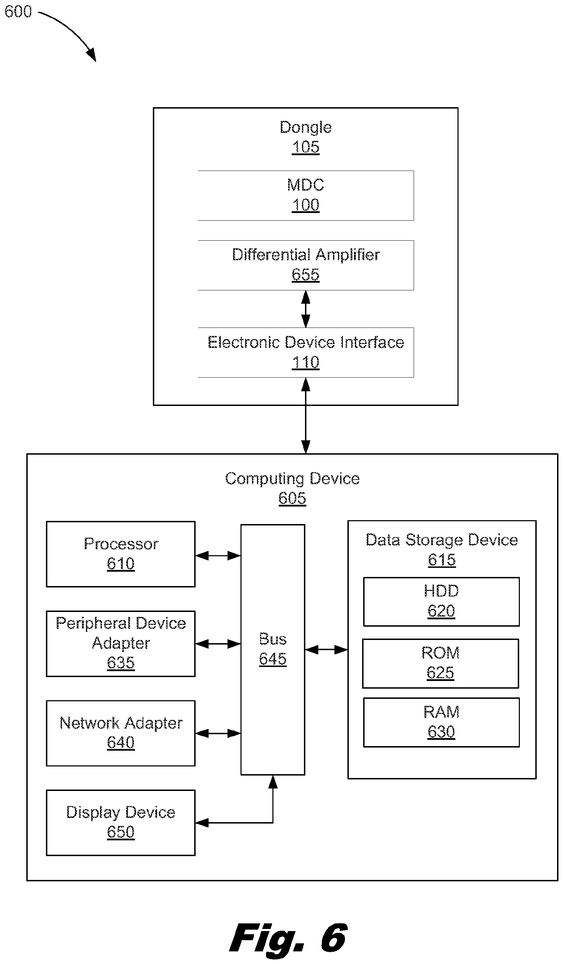

Fig. 6 is a block diagram of a microfluidic diagnostic chip system (600) according to one example of the principles described herein. The system (600) includes a computing device (605) and a cassette (105) selectively electrically coupled to the computing device (605). The cassette (105) includes a MDC (100) and an electronic device interface (110) as described above in connection with Fig. 1. In one example, the cassette (105) may be communicatively coupled to the computing device (505) via a USB connector.

The computing device (605) includes various hardware components. Among these hardware components may be a number of processors (610), a number of data storage devices (615), a number of peripheral device adapters (635), a number of network adapters (640), and a display device (650). These hardware components may be interconnected through the use of a number of busses (645) and/or network connections. In one example, the processor (610), data storage device (615), peripheral device adapters (635), and network adapter (640) may be communicatively coupled via a bus (645).

The processor (610) may include the hardware architecture to retrieve executable code from the data storage device (615) and execute the executable code. The executable code may, when executed by the processor (610), cause the processor (610) to implement at least the functionality of receiving a number of electrical signals from the MDC (100) via the electronic device interface (110) and the peripheral device adapter (635), according to the methods of the present specification described herein. In the course of executing code, the processor (610) may receive input from and provide output to a number of the remaining hardware units.

The data storage device (615) may store data such as executable program code that is executed by the processor (610) or other processing device. The data storage device (610) may specifically store computer code representing a number of applications that the processor (610) executes to implement at least the functionality described herein.

The data storage device (615) may include various types of memory modules, including volatile and nonvolatile memory. For example, the data storage device (615) of the present example includes Random Access Memory (RAM) (630), Read Only Memory (ROM) (625), and Hard Disk Drive (HDD) memory (620). Many other types of memory may also be utilized, and the present specification contemplates the use of many varying type(s) of memory in the data storage device (615) as may suit a particular application of the principles described herein. In certain examples, different types of memory in the data storage device (615) may be used for different data storage needs. For example, in certain examples the processor (610) may boot from Read Only Memory (ROM) (625), maintain nonvolatile storage in the Hard Disk Drive (HDD) memory (620), and execute program code stored in Random Access Memory (RAM) (630).

Generally, the data storage device (615) may include a computer readable medium, a computer readable storage medium, or a non-transitory computer readable medium, among others. For example, the data storage device (615) may be, but not limited to, an electronic, magnetic, optical, electromagnetic, infrared, or semiconductor system, apparatus, or device, or any suitable combination of the foregoing. More specific examples of the computer readable storage medium may include, for example, the following: an electrical connection having a number of wires, a portable computer diskette, a hard disk, a random access memory (RAM), a read-only memory (ROM), an erasable programmable read-only memory (EPROM or Flash memory), a portable compact disc read-only memory (CD-ROM), an optical storage device, a magnetic storage device, or any suitable combination of the foregoing. In the context of this document, a computer readable storage medium may be any tangible medium that can contain, or store computer usable program code for use by or in connection with an instruction execution system, apparatus, or device. In another example, a computer readable storage medium may be any non-transitory medium that can contain, or store a program for use by or in connection with an instruction execution system, apparatus, or device.

The hardware adapters (635, 640) in the computing device (605) enable the processor (610) to interface with various other hardware elements, external and internal to the computing device (610). For example, the peripheral device adapters (635) may provide an interface to input/output devices, such as, for example, display device (650), a mouse, or a keyboard. The peripheral device adapters (635) may also provide access to other external devices such as an external storage device, a number of network devices such as, for example, servers, switches, and routers, client devices, other types of computing devices, and combinations thereof.

The display device (650) may be provided to allow a user of the computing device (605) to interact with and implement the functionality of the computing device (605). The peripheral device adapters (635) may also create an interface between the processor (610) and the display device (650), a printer, or other media output devices. The network adapter (640) may provide an interface to other computing devices within, for example, a network, thereby enabling the transmission of data between the computing device (605) and other devices located within the network.

Aspects of the present system and method are described herein with reference to flowchart illustrations and/or block diagrams of methods, apparatus (systems) and computer program products according to examples of the principles described herein. Each block of the flowchart illustrations and block diagrams, and combinations of blocks in the flowchart illustrations and block diagrams, may be implemented by computer usable program code. The computer usable program code may be provided to a processor of a general purpose computer, special purpose computer, or other programmable data processing apparatus to produce a machine, such that the computer usable program code, when executed via, for example, the processor (610) of the computing device (605) or other programmable data processing apparatus, implement the functions or acts specified in the flowchart and/or block diagram block or blocks. In one example, the computer usable program code may be embodied within a computer readable storage medium; the computer readable storage medium being part of the computer program product. In one example, the computer readable storage medium is a non-transitory computer readable medium.

The specification and figures describe a diagnostic chip that removes background noise in an impedance signal of an analyte. This diagnostic chip may, for example, allow for a better cell impedance signal to noise ratio. Additionally the MDC may, for example, allow for high accuracy of cell detection and counting on the microfluidic scale.

The preceding description has been presented to illustrate and describe examples of the principles described. This description is not intended to be exhaustive or to limit these principles to any precise form disclosed. Many modifications and variations are possible in light of the above teaching.

## Claims

1. A microfluidic diagnostic chip (100), comprising:
a number of input microfluidic channels (205);
a number of output microfluidic channels (210);
a number of interposing microfluidic channels (215) fluidly coupled at one end to at least one input microfluidic channel and at a second end to at least one output microfluidic channel; and
first and second microfluidic sensors (220) within the number of interposing microfluidic channels;
wherein the microfluidic diagnostic chip is to compare output from the first microfluidic sensor sensing a fluid comprising an analyte to output from the second microfluidic sensor sensing the fluid lacking the analyte,
wherein the number of interposing microfluidic channels comprises a plurality of interposing microfluidic channels with the first and second microfluidic sensors each in a separate interposing microfluidic channel, and
wherein the microfluidic diagnostic chip further comprises a thin film resistor to pump the fluid through the input, output, and interposing microfluidic channels, wherein the thin film resistor is encapsulated with a passive film and wherein the film resistor and passive film are encapsulated with a cavitation film,
the microfluidic diagnostic chip further comprising: a blocking device (225) to prevent particles of the analyte within the fluid from being sensed by the second microfluidic sensor;
wherein there is a differential amplifier (305) communicatively coupled to or within the microfluidic diagnostic chip to receive signals from the first and second microfluidic sensors (220) and compute a differential output.

2. The microfluidic diagnostic chip (100) of claim 1, wherein the first and second microfluidic sensors (220) measure impedance of the first fluid comprising the analyte and the second fluid without the analyte in real time.

3. The microfluidic diagnostic chip (100) of claim 1 or claim 2, wherein the blocking device (225) comprises a number of particle blocking pillars (225) around the second microfluidic sensor (220).

4. A method of removing background noise from an impedance value of an analyte in a microfluidic diagnostic chip (100) comprising a number of input microfluidic channels (205), a number of output microfluidic channels (210), a plurality of interposing microfluidic channels (215) fluidly coupled at one end to at least one input microfluidic channel and at a second end to at least one output microfluidic channel, with first and second microfluidic sensors (220) each in a separate interposing microfluidic channel, wherein the microfluidic diagnostic chip further comprises a thin film resistor to pump the fluid through the input, output, and interposing microfluidic channels, wherein the thin film resistor is encapsulated with a passive film and wherein the film resistor and passive film are encapsulated with a cavitation film, the microfluidic diagnostic chip further comprising: a blocking device (225) to prevent particles of the analyte within the fluid from being sensed by the second microfluidic sensor,
the method comprising:
measuring, with the first sensor, the impedance value of a fluid comprising an analyte;
measuring, with the second sensor, the impedance value of the fluid without the analyte; and
comparing, with a differential amplifier, the impedance value detected by the first sensor to the impedance value detected by the second sensor.

## Patentansprüche

1. Mikrofluidischer Diagnosechip (100), der Folgendes umfasst:
eine Anzahl mikrofluidischer Eingabekanäle (205);
eine Anzahl mikrofluidischer Ausgabekanäle (210);
eine Anzahl dazwischenliegender mikrofluidischer Kanäle (215), die an einem Ende mit wenigstens einem mikrofluidischen Eingabekanal und an einem zweiten Ende mit wenigstens einem mikrofluidischen Ausgabekanal fluidisch gekoppelt sind; und
einen ersten und einen zweiten mikrofluidischen Sensor (220) innerhalb der Anzahl dazwischenliegender mikrofluidischer Kanäle;
wobei der mikrofluidische Diagnosechip eine Ausgabe aus dem ersten mikrofluidischen Sensor, der ein Fluid erfasst, das einen Analyten umfasst, mit der Ausgabe aus dem zweiten mikrofluidischen Sensor, der das Fluid erfasst, dem der Analyt fehlt, vergleichen soll,
wobei die Anzahl der dazwischenliegenden mikrofluidischen Kanäle mehrere dazwischenliegende mikrofluidische Kanäle umfasst, wobei der erste und der zweite mikrofluidische Sensor jeweils in einem separaten dazwischenliegenden mikrofluidischen Kanal sind, und
wobei der mikrofluidische Diagnosechip ferner einen Dünnschichtwiderstand umfasst, um das Fluid durch die Eingabe-, die Ausgabe- und die dazwischenliegenden mikrofluidischen Kanäle zu pumpen, wobei der Dünnschichtwiderstand mit einer Passivschicht eingekapselt ist und wobei der Schichtwiderstand und die Passivschicht mit einer Kavitationsschicht eingekapselt sind,
wobei der mikrofluidische Diagnosechip ferner Folgendes umfasst:
eine Blockiervorrichtung (225), um zu verhindern, dass Partikel des Analyten innerhalb des Fluids von dem zweiten mikrofluidischen Sensor erfasst werden;
wobei es einen Differenzverstärker (305) gibt, der mit dem oder innerhalb des mikrofluidischen Diagnosechips kommunikativ gekoppelt ist, um Signale von dem ersten und dem zweiten mikrofluidischen Sensor (220) zu empfangen und eine Differenzausgabe zu berechnen.

2. Mikrofluidischer Diagnosechip (100) nach Anspruch 1, wobei der erste und der zweite mikrofluidische Sensor (220) eine Impedanz des ersten Fluids, das den Analyten umfasst, und des zweiten Fluids, ohne den Analyten, in Echtzeit messen.

3. Mikrofluidischer Diagnosechip (100) nach Anspruch 1 oder 2, wobei die Blockiervorrichtung (225) eine Anzahl partikelblockierender Säulen (225) um den zweiten mikrofluidischen Sensor (220) herum umfasst.

4. Verfahren zum Entfernen von Hintergrundrauschen aus einem Impedanzwert eines Analyten in einem mikrofluidischen Diagnosechip (100), umfassend eine Anzahl mikrofluidischer Eingabekanäle (205), eine Anzahl mikrofluidischer Ausgabekanäle (210), mehrere dazwischenliegende mikrofluidische Kanäle (215), die an einem Ende mit wenigstens einem mikrofluidischen Eingabekanal und an einem zweiten Ende mit wenigstens einem mikrofluidischen Ausgabekanal fluidisch gekoppelt sind, wobei der erste und der zweite mikrofluidische Sensor (220) jeweils in einem separaten dazwischenliegenden mikrofluidischen Kanal sind, wobei der mikrofluidische Diagnosechip ferner einen Dünnschichtwiderstand umfasst, um das Fluid durch die Eingabe-, die Ausgabe- und die dazwischenliegenden mikrofluidischen Kanäle zu pumpen, wobei der Dünnschichtwiderstand mit einer Passivschicht eingekapselt ist und wobei der Schichtwiderstand und die Passivschicht mit einer Kavitationsschicht eingekapselt sind, wobei der mikrofluidische Diagnosechip ferner Folgendes umfasst:
eine Blockiervorrichtung (225), um zu verhindern, dass Partikel des Analyten innerhalb des Fluids von dem zweiten mikrofluidischen Sensor erfasst werden, wobei das Verfahren Folgendes umfasst:
Messen des Impedanzwerts eines Fluids, das einen Analyten umfasst, mit dem ersten Sensor;
Messen des Impedanzwerts des Fluids ohne den Analyten mit dem zweiten Sensor; und
Vergleichen des von dem ersten Sensor festgestellten Impedanzwerts mit dem von dem zweiten Sensor festgestellten Impedanzwert mit einem Differenzverstärker.

## Revendications

1. Puce de diagnostic microfluidique (100), comprenant :
un certain nombre de canaux microfluidiques d'entrée (205) ;
un certain nombre de canaux microfluidiques de sortie (210) ;
un certain nombre de canaux microfluidiques d'interposition (215) accouplés fluidiquement à une extrémité à au moins un canal microfluidique d'entrée et à une seconde extrémité à au moins un canal microfluidique de sortie ; et
des premier et second capteurs microfluidiques (220) dans le nombre de canaux microfluidiques d'interposition ;
la puce de diagnostic microfluidique devant comparer la sortie du premier capteur microfluidique détectant un fluide comprenant un analyte à sortir du second capteur microfluidique détectant le fluide dépourvu de l'analyte, le nombre de canaux microfluidiques d'interposition comprenant une pluralité de canaux microfluidiques d'interposition avec les premier et second capteurs microfluidiques, chacun dans un canal microfluidique d'interposition séparé, et la puce de diagnostic microfluidique comprenant en outre une résistance à film mince pour pomper le fluide à travers l'entrée, la sortie et les canaux microfluidiques d'interposition, la résistance à film mince est encapsulée à l'aide d'un film passif et la résistance de film et le film passif étant encapsulés à l'aide d'un film de cavitation, la puce de diagnostic microfluidique comprenant en outre :
un dispositif de blocage (225) pour empêcher les particules de l'analyte présentes dans le fluide d'être détectées par le second capteur microfluidique ;
un amplificateur différentiel (305) couplé en communication à ou à l'intérieur de la puce de diagnostic microfluidique pour recevoir des signaux des premier et second capteurs microfluidiques (220) et calculer une sortie différentielle.

2. Puce de diagnostic microfluidique (100) selon la revendication 1, les premier et second capteurs microfluidiques (220) mesurant l'impédance du premier fluide comprenant l'analyte et du second fluide sans l'analyte en temps réel.

3. Puce de diagnostic microfluidique (100) selon l'une quelconque des revendications 1 ou 2, le dispositif de blocage (225) comprenant un certain nombre de piliers de blocage de particules (225) autour du second capteur microfluidique (220).

4. Procédé pour éliminer le bruit de fond d'une valeur d'impédance d'un analyte dans une puce de diagnostic microfluidique (100) comprenant un certain nombre de canaux microfluidiques d'entrée (205), un certain nombre de canaux microfluidiques de sortie (210), une pluralité de canaux microfluidiques d'interposition (215) accouplés fluidiquement à une extrémité à au moins un canal microfluidique d'entrée et à une seconde extrémité à au moins un canal microfluidique de sortie, avec des premier et second capteurs microfluidiques (220) chacun dans un canal microfluidique d'interposition séparé, la puce de diagnostic microfluidique comprenant en outre une résistance à film mince pour pomper le fluide à travers les canaux microfluidiques d'entrée, de sortie et d'interposition, la résistance à film mince étant encapsulée à l'aide d'un film passif et la résistance à film et le film passif étant encapsulés à l'aide d'un film de cavitation, la puce de diagnostic microfluidique comprenant en outre :
un dispositif de blocage (225) pour empêcher les particules de l'analyte présentes dans le fluide d'être détectées par le second capteur microfluidique, le procédé comprenant :
la mesure, à l'aide du premier capteur, de la valeur d'impédance d'un fluide comprenant un analyte ;
la mesure, à l'aide du second capteur, de la valeur d'impédance du fluide sans l'analyte ; et
la comparaison, à l'aide d'un amplificateur différentiel, de la valeur d'impédance détectée par le premier capteur à la valeur d'impédance détectée par le second capteur.
